# FASCICULE DE BREVET EUROPEEN

(11) **EP 2 058 520 B1**
(45) Date de publication et mention de la délivrance du brevet: **14.07.2010**
(21) Numéro de dépôt: 08167020.0
(22) Date de dépôt: 20.10.2008
(51) Int. Cl.: F04C 28/28, F04C 23/02, F04B 49/02

(54) **Installation de secours de production de vide et système de production de vide correspondant.**
Hilfsanlage zum Herstellen eines Vakuums und entsprechendes System zum Herstellen eines Vakuums
Backup vacuum-production installation and corresponding vacuum-production system.

(30) Priorité: 07.11.2007 FR 0707821
(43) Date de publication de la demande: 13.05.2009
(73) Titulaire: Mil'S, 69740 Genas (FR)
(72) Inventeur: Gentien, Dominique, 69004 Lyon (FR)
(74) Mandataire: Maureau, Philippe

(56) Documents cités:
- DE-A1- 2 555 823
- FR-A- 2 789 189
- FR-A- 2 806 173

## Description

L'invention concerne une installation de secours de production de vide ainsi qu'un système de production de vide correspondant.

Une installation de secours de production de vide est connue du document DE 25 55 823 que divulgue les caractéristiques du préambule de la revendication 1.

Une installation de production de vide est notamment utilisée afin de produire du vide à l'intérieur d'un réseau de vide d'un hôpital ou d'une clinique. Le réseau de vide est relié à différents points d'aspiration se trouvant dans des chambres ou dans des blocs opératoires.

Le vide est utilisé notamment pour nettoyer les champs opératoires ou dégager les voies respiratoires d'un patient et constitue donc un élément vital.

En cas de perte d'alimentation en vide, par exemple lors d'une panne électrique, le temps de réaction doit être extrêmement court afin de ne pas mettre en danger la vie des patients ayant besoin de soins nécessitant du vide.

Le vide ayant une capacité de stockage très limitée, l'autonomie du réseau de vide est restreinte.

De manière classique, les installations de production de vide utilisent une pompe à vide actionnée électriquement. Ainsi, en cas de panne électrique, les installations de production sont secourues par un groupe électrogène.

Il peut toutefois arriver que le groupe électrogène soit sous-dimensionné et ne permette alors pas de pouvoir actionner la ou les pompes à vide motorisées ou encore que le groupe électrogène ne démarre pas du fait d'une longue période d'inactivité ou d'un défaut de fonctionnement.

Afin de remédier à ces inconvénients, l'invention propose une installation de secours de production de vide permettant de remplacer les groupes électrogènes classiques, tout en offrant une autonomie énergétique suffisante pour permettre d'assurer la production de vide avant le rétablissement de l'installation principale.

A cet effet, l'invention concerne une installation de secours de production de vide, destinée à être raccordée à un réseau de vide d'un hôpital en cas de panne d'une installation principale de production de vide, **caractérisée en ce qu**'elle comporte une pompe à vide comportant une entrée d'aspiration destinée à être raccordée au réseau de vide, la pompe à vide étant couplée à un moteur pneumatique raccordé à des moyens de stockage d'air conçus pour délivrer de l'air en cas de panne de manière à actionner le moteur pneumatique.

L'air stocké permet ainsi d'actionner un moteur pneumatique, actionnant lui-même la pompe à vide permettant de fournir du vide dans le réseau de vide.

Le dimensionnement des moyens de stockage permet d'ajuster la durée de fonctionnement de l'installation de secours.

L'installation de secours selon l'invention permet ainsi de s'affranchir d'une éventuelle panne d'électricité en utilisant des éléments mécaniques fiables et simples tels que le moteur pneumatique.

Selon une caractéristique de l'invention, l'installation comporte un élément d'aspiration de type venturi possédant une branche d'écoulement principale, raccordée à la sortie de refoulement du moteur pneumatique, et une branche d'aspiration raccordée à la pompe à vide.

La pompe à vide et le venturi forment ainsi respectivement un premier et un second étages d'aspiration. Cette caractéristique permet d'améliorer le rendement de l'installation.

Avantageusement, l'installation comporte des moyens de réglage du débit d'air délivré au moteur pneumatique, par exemple une vanne commandée, disposés sur une conduite d'alimentation reliant les moyens de stockage d'air au moteur pneumatique, les moyens de réglage étant commandés en fonction de la pression à l'intérieur du réseau de vide.

Il est ainsi possible de démarrer ou d'arrêter la production de vide en fonction de valeurs seuils de pression à l'intérieur du réseau de vide, c'est-à-dire uniquement en fonction des besoins. L'autonomie de l'installation de secours est donc augmentée.

Selon une possibilité de l'invention, les moyens de réglage comportent un premier et un second vacuostats coopérant avec une mémoire pneumatique, les moyens de réglage étant conçus pour ouvrir une vanne de réglage du débit d'air dirigé vers le moteur pneumatique lorsque la pression à l'intérieur du réseau de vide est supérieure à une valeur haute limite de pression et pour fermer la vanne de réglage lorsque la pression à l'intérieur du réseau de vide est inférieure à une valeur basse limite de pression.

Préférentiellement, la valeur haute limite de pression est de l'ordre de -300 mbar et la valeur basse limite de pression est de l'ordre de -650 mbar.

Ainsi, lorsque la pression à l'intérieur du réseau de vide est supérieur à -300 mbar, c'est-à-dire en cas de vide faible, l'installation est mise en marche de manière à produire du vide et donc de manière à diminuer la pression dans le réseau de vide. Lorsque la pression dans le réseau de vide est inférieure à -650 mbar, l'installation s'arrête.

L'alarme réseau dite « vide faible », présente de manière classique sur un réseau de vide d'un hôpital ou d'une clinique, est réglée à -370 mbar. Ainsi, le fonctionnement de l'installation de secours reste visible par l'alarme réseau de vide faible, ce qui permet d'alerter le personnel du disfonctionnement global de l'installation. Toutefois, un vide de -300 mbar ne met pas en danger les fonctions vitales devant être assurées à l'aide du vide.

En outre, bien qu'une installation classique permette de produire un vide régulé entre -650 mbar et -850 mbar, de telles valeurs importantes de vide ne sont pas recherchées car elles nécessite une dépense d'énergie importante, incompatible avec une autonomie énergétique suffisante de l'installation de secours. Néanmoins, comme indiqué précédemment, le fonctionnement entre - 300 mbar et -650 mbar permet d'assurer l'ensemble des fonctions vitales de l'hôpital devant être secourues.

Selon une caractéristique de l'invention, les moyens de réglage sont alimentés en air par les moyens de stockage d'air et/ou par un réseau d'air distinct.

L'invention concerne en outre un système de production de vide comportant une installation principale de production de vide, destinée à produire du vide lors du fonctionnement normal du système, de manière à fournir du vide dans un réseau de vide, **caractérisée en ce qu**'il comporte une installation de secours selon l'invention, conçue pour produire du vide dans le réseau de vide, en cas de panne de l'installation principale de production de vide.

De toute façon, l'invention sera bien comprise à l'aide de la description qui suit, en référence au dessin schématique annexé représentant, à titre d'exemples, deux formes de réalisation de cette installation de secours de production de vide.
Figure 1 est une vue schématique d'une première forme de réalisation de l'invention ;
Figure 2 est une vue correspondant à la figure 1, d'une seconde forme de réalisation de l'invention ;
Figures 3 à 7 sont des vues schématiques des moyens de réglage du débit d'air délivré au moteur pneumatique, dans plusieurs phases successives ;
Figure 8 est un diagramme représentant, en parallèle, et en fonction de la phase des moyens de réglage, l'état des divers éléments formant les moyens de réglage ainsi que la valeur de la pression dans le réseau de vide ;
Figure 9 est une vue en coupe du venturi.

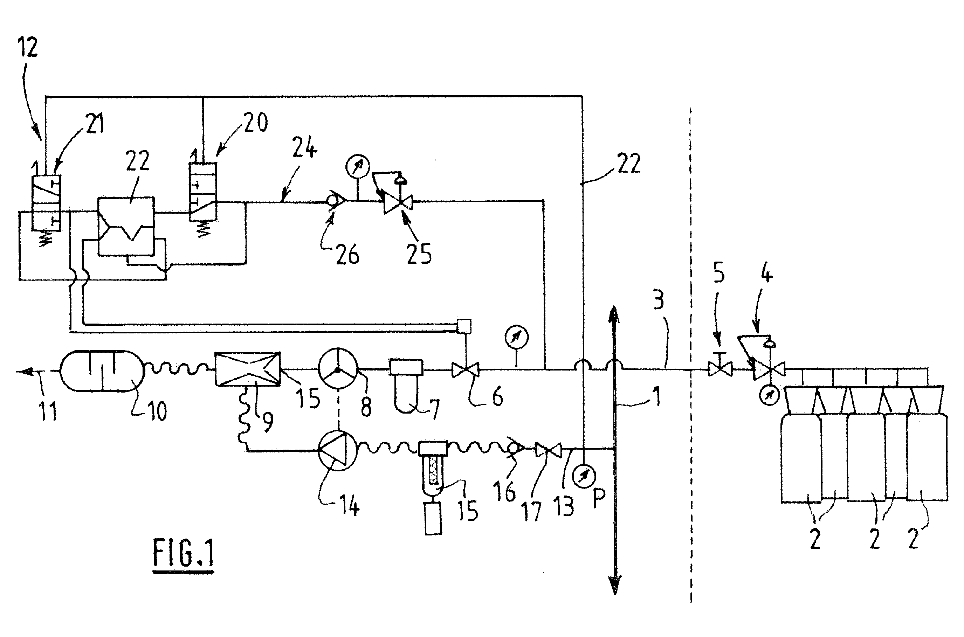

La figure 1 représente une première forme de réalisation de l'installation de secours de production de vide, raccordée à un réseau de vide 1 d'un hôpital en cas de panne d'une installation principale de production de vide (non représentée).

Cette installation de secours comporte une série de bouteilles 2 contenant de l'air haute pression de qualité non médicale.

Plus particulièrement, l'installation comporte neuf bouteilles d'une contenance de 50 litres, comportant de l'air comprimé à une pression de 200 bars. Les bouteilles ont donc une autonomie de 90 m³.

Une première conduite 3 reliée aux bouteilles au niveau de son extrémité amont, est équipée successivement, de l'amont vers l'aval, d'un détendeur 4, d'une vanne d'isolement manuelle 5, d'une vanne commandée 6, d'un lubrificateur 7, d'un moteur pneumatique 8, d'un système venturi 9 et d'un silencieux 10. L'extrémité aval de la conduite 3 constitue une extrémité de refoulement 11 de l'air vers l'extérieur.

La vanne 6 est commandée par l'intermédiaire de moyens de réglage 12 du débit d'air délivré au moteur pneumatique 8, dont la structure et le fonctionnement sont décrits ci-après.

Une seconde conduite 13, formant une conduite de fourniture de vide, relie le réseau de vide 1 à l'entrée d'aspiration d'une pompe à vide 14, couplée au moteur pneumatique 8, par l'intermédiaire d'un filtre anti-bactérien 15, d'un clapet anti-retour 16 et d'une vanne 17..

La pompe à vide 14 utilisée dans l'invention est une pompe à palettes lubrifiées et comporte une structure modifiée par rapport à une pompe à vide classique. Les modifications apportées portent sur le fait que tous les accessoires classiques nécessaires au refroidissement de la pompe (ventilateur, turbine) ont été supprimés de manière à limiter les frottements, et donc à augmenter le rendement de la pompe à vide.

De plus, la sortie de refoulement du moteur pneumatique 8 est reliée à une entrée 15 du venturi 9.

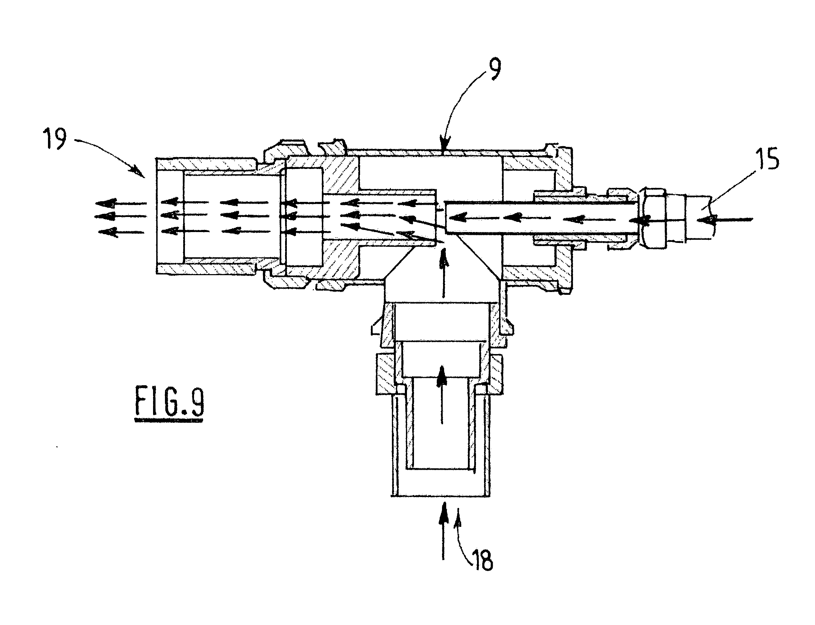

La structure du venturi 9 est représentée plus particulièrement à la figure 9. Le venturi 9 comporte ainsi, outre l'entrée 15, une entrée d'aspiration 18 reliée à la pompe à vide 14 et une sortie de refoulement 19 tournée du côté de l'extrémité de refoulement 11 de la première conduite 3. De cette manière, lorsque l'air refoulé par le moteur pneumatique 8 est injecté dans l'entrée 15, le venturi produit du vide au niveau de son entrée d'aspiration 18.

Ainsi, en cas de panne de l'installation principale, l'air des bouteilles 2 circule en direction du moteur pneumatique 8 de manière à actionner la pompe à vide 14 et ainsi produire du vide dans le réseau de vide 1. L'air refoulé par le moteur pneumatique 8 est injecté dans le venturi 9, ce qui produit du vide au niveau de son entrée d'aspiration 18, c'est-à-dire en aval de la pompe à vide 14.

L'installation comporte ainsi un système de production de vide à double étages, un premier étage de production de vide étant formé par la pompe à vide 14, un second étage de production de vide étant formé par le venturi 9.

La vanne de réglage commandée 6 permet d'ajuster le débit d'air dirigé vers le moteur pneumatique 8 et ainsi de régler la production de vide.

Le silencieux 10 disposé en partie aval de la conduite 3 permet de limiter les nuisances sonores de l'installation de secours.

La structure et le fonctionnement des moyens de réglage 12 permettant la commande de la vanne 6 vont maintenant être décrits plus en détail.

Dans la première forme de réalisation représentée à la figure 1, les moyens de réglage 12 comportent un premier et un second vacuostats 20, 21, couplés à une mémoire pneumatique 22.

L'actionnement des vacuostats 20, 21 est commandé en fonction de la pression p à l'intérieur du réseau de vide 1, par l'intermédiaire d'un piquage 22. L'alimentation 24 des vacuostats 20, 21 est prise sur la réserve d'air moteur, c'est-à-dire sur la conduite 3, en amont de la vanne commandée 6, par l'intermédiaire d'un détendeur 25 réglé à 4 bars et d'un clapet anti-retour 26.

Les moyens de réglage 12 permettent d'ouvrir la vanne commandée 6, c'est-à-dire d'assurer la production de vide, lorsque la pression p à l'intérieur du réseau de vide 1 est supérieur à -300 mbar et pour fermer la vanne commandée 6, c'est-à-dire arrêter la production de vide, lorsque la pression p à l'intérieur du réseau de vide 1 est inférieure à -650 mbar.

Proche de leur pression d'enclenchement, les vacuostats 20,21 consomment de l'air, nécessaire à leur bon fonctionnement. Lors des phases d'attente, le réseau de vide de l'hôpital étant entre -650 et -850 mb, le vacuostat 21 (-600 mb) est particulièrement potentiellement concerné par cette consommation d'air propre à son fonctionnement. Par conséquent, son alimentation en air est coupée tant que le vacuostat 20 n'a pas activité la mémoire 22.

Toutefois, l'ensemble de la partie pneumatique présente un taux de fuite d'air qu'il ne faut pas négliger.

La forme de réalisation représentée à la figure 1 présente l'inconvénient de consommer de l'air des bouteilles 2 pendant les phases d'attente. Toutefois l'air consommé n'est pas de qualité médicale, ce qui diminue les coûts de fonctionnement d'une telle installation. En outre, le taux de fuite, c'est-à-dire l'air consommé pendant les phases d'attente, est très faible. Il est de l'ordre de 8 m³ pour 6 mois, soit moins d'une bouteille d'une contenance de 50 L à 200 bars.

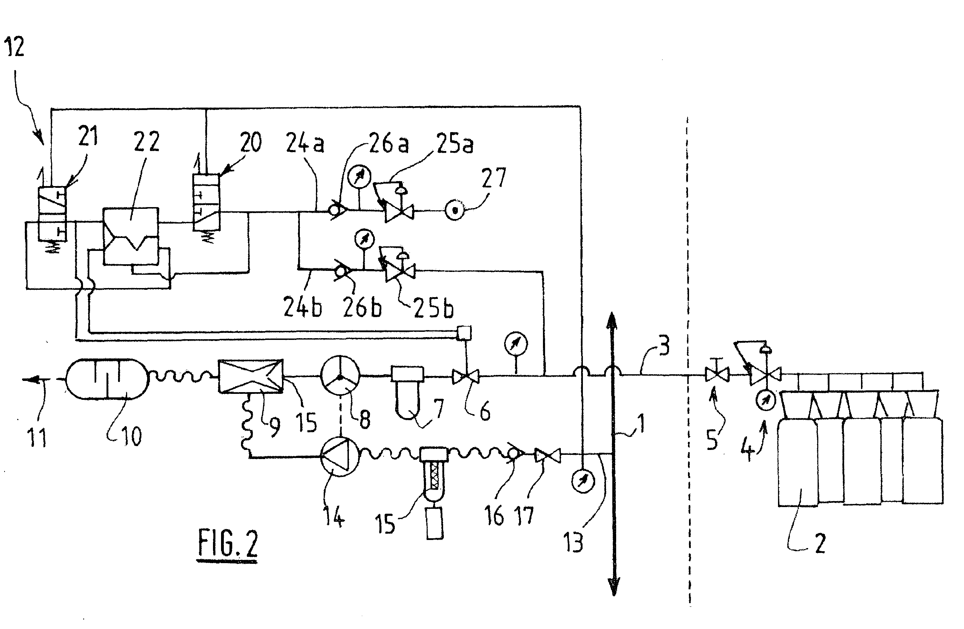

Dans la forme de réalisation représentée à la figure 2, les bouteilles 2 sont remplies à l'aide d'air de qualité médicale.

Dans cette forme de réalisation, les vacuostats 20, 21 et la mémoire 22 sont alimentés par deux sources d'alimentation distinctes 24a, 24b.

La première source d'alimentation 24a, avec un détendeur 25a réglé à 4 bars, est prise sur un réseau général d'air comprimé de qualité médicale 27.

La seconde source d'alimentation, avec un détendeur 25b réglé à 3 bars, est prise comme précédemment, sur la réserve d'air moteur, c'est-à-dire sur la conduite 3, en amont de la vanne commandée 6.

En fonctionnement normal, l'alimentation des moyens de réglage est assurée par la première alimentation 24a, dont la pression est plus élevée.

En cas de rupture d'alimentation sur le réseau général d'air comprimé de l'hôpital, alors la seconde alimentation 24b prend le relais sur la première alimentation 24a lors de la chute de pression correspondante.

L'agencement des vacuostats 20, 21 et de la mémoire 22 ainsi que la commande des vacuostats restent les mêmes que dans la première forme de réalisation.

Cette solution a pour avantage de ne pas consommer d'air des bouteilles 2 pendant les phases d'attente lorsque l'alimentation est réalisée de façon normale par le biais du réseau général 27 de l'hôpital. Toutefois, les coûts engendrés par le fonctionnement d'une telle installation sont importants car l'air permettant l'alimentation des moyens de réglage 12 est de qualité médicale.

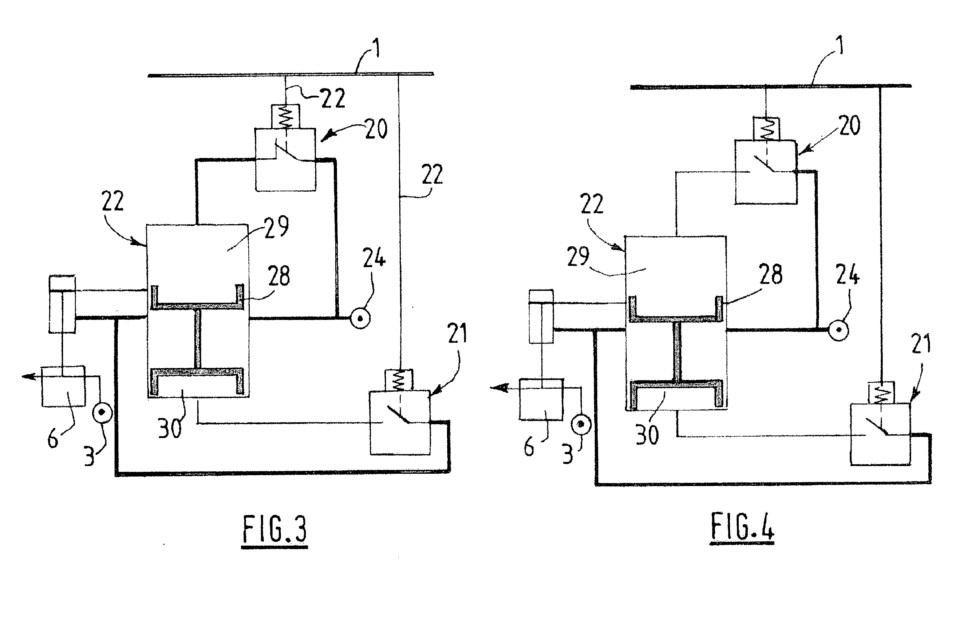

Le fonctionnement des moyens de réglage 12 est mieux décrit ci-après en référence aux figures 3 à 7. Sur ces figures, les mêmes références désignent les mêmes éléments, la schématisation des éléments étant toutefois différente.

Comme vu précédemment, la commande des vacuostats 20, 21 est assurée en fonction de la pression p dans le réseau de vide 1 de l'hôpital.

Le premier vacuostat 20 est réglé à une pression seuil de -300 mbar, le second vacuostat 21 étant réglé à une pression seuil de -600 mbar. Chaque vacuostat 20, 21 comporte un hystérésis réglé à 50 mbar.

La mémoire 22 est structurellement formée par un piston 28 dont la position peut être maintenue ou modifiée en fonction de la pression dans une première et une seconde chambres 29, 30 séparées par le piston 28.

La mémoire 22 est reliée à la vanne commandée 6.

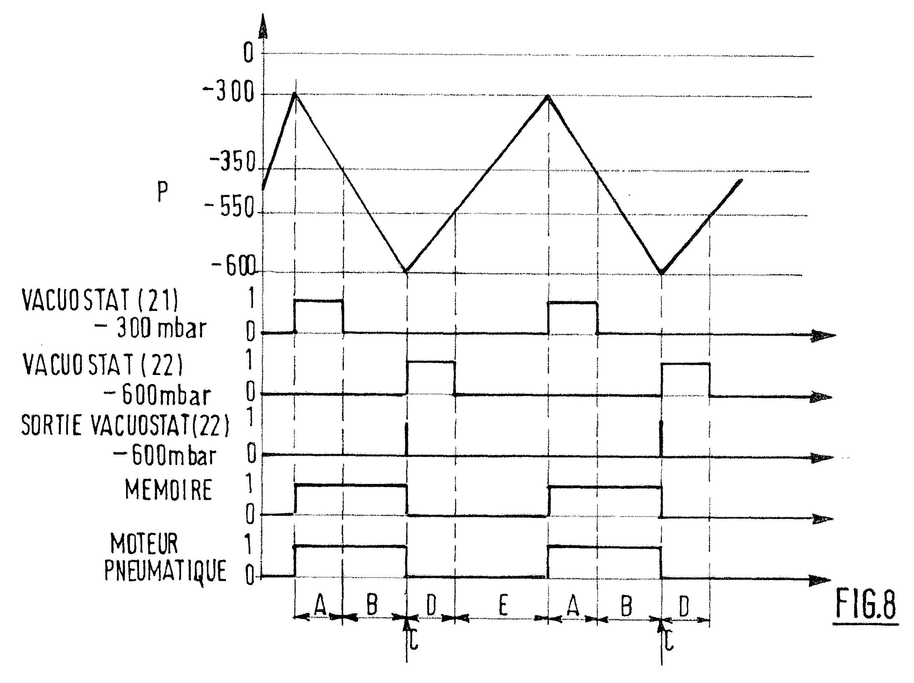

Dans une première phase représentée à la figure 3 et référencée A sur le diagramme de phases de la figure 8, le vide à l'intérieur du réseau 1 est faible, la pression p dans le réseau de vide 1 étant comprise entre -300 mbar et -350 mbar. Le piston 28 de la mémoire 22 est dans une première position, dénommée ci-après pour des raisons de commodité « position basse ». En outre, la vanne 6 est ouverte, ce qui signifie que l'air peut circuler en direction du moteur pneumatique 8 de manière à actionner la pompe à vide 14. Le vacuostat 21 est alors alimenté en air afin de surveiller la pression du réseau de vide.

Dans cette phase, le premier vacuostat 20 est activé, c'est-à-dire à l'état « 1 » permettant le passage d'air. Le second vacuostat 21 est désactivé, c'est-à-dire à l'état « 0 » ne permettant pas le passage d'air.

L'air issu de l'alimentation 24 entre donc dans la chambre haute 29 de la mémoire 22 de manière à maintenir le piston 28 en position basse.

Ainsi, lorsque le vide à l'intérieur du réseau de vide 1 de l'hôpital est faible, l'installation produit du vide tant qu'un niveau faible de vide est détecté par les vacuostats 20, 21.

Suite à la production de vide, la pression p à l'intérieur du réseau de vide 1 chute sous le seuil de -350 mbar. Dans cette phase, référencée B sur le diagramme et représenté à la figure 4, le premier vacuostat 20 est désactivé, le second vacuostat 21 restant désactivé. La désactivation du premier vacuostat 20 intervient à -350 mbar (et non pas -300 mbar) car le cycle de fonctionnement d'un vacuostat se présente, comme cela est connu en soi, sous la forme d'un hystérésis. La valeur de l'hystérésis est réglé à 50 mbar, comme vu précédemment.

Dans cette phase, la position du piston 28 est maintenue et la vanne commandée 6 reste ouverte de sorte que du vide continue d'être produit par l'installation. La pression p dans le réseau de vide 1 continue donc de chuter jusqu'à atteindre une pression -600 mbar.

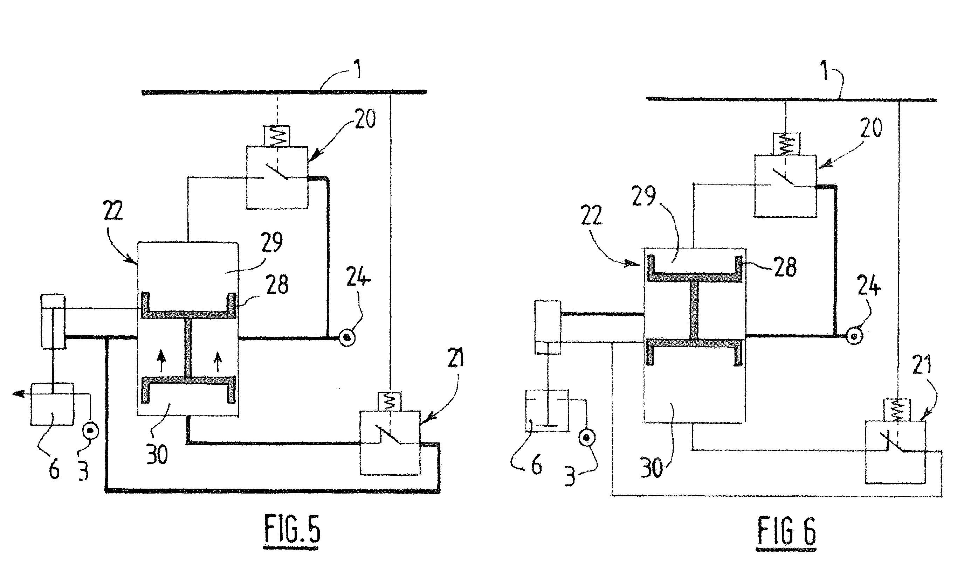

La phase suivante est référencée C sur le diagramme et est représentée à la figure 5. Lors de cette phase, le second vacuostat 21 est activé, le premier vacuostat 20 restant désactivé. L'air sous pression issu de l'alimentation 24 alimente donc une seconde chambre 30 de la mémoire 22, opposée à la première 29, de sorte que le piston 28 est déplacé en position haute.

Lors de son déplacement vers le haut, le piston 28 actionne la commande de la vanne 6 de manière à fermer cette dernière et en même temps le piston 28 coupe l'alimentation en air du vacuostat 21, donc sa sortie s'en trouve désactivée.

L'installation atteint alors la phase référencée D sur le diagramme et représentée à la figure 6

La fermeture de la vanne 6 coupe l'alimentation du moteur pneumatique 8. Ainsi, la production de vide est arrêtée lorsqu'un niveau de vide important est détecté.

Le vide va ainsi être « consommé » progressivement sans que la pompe à vide 14 et le moteur pneumatique 8 ne fonctionnent, faisant ainsi remonter la pression p à l'intérieur du réseau de vide 1.

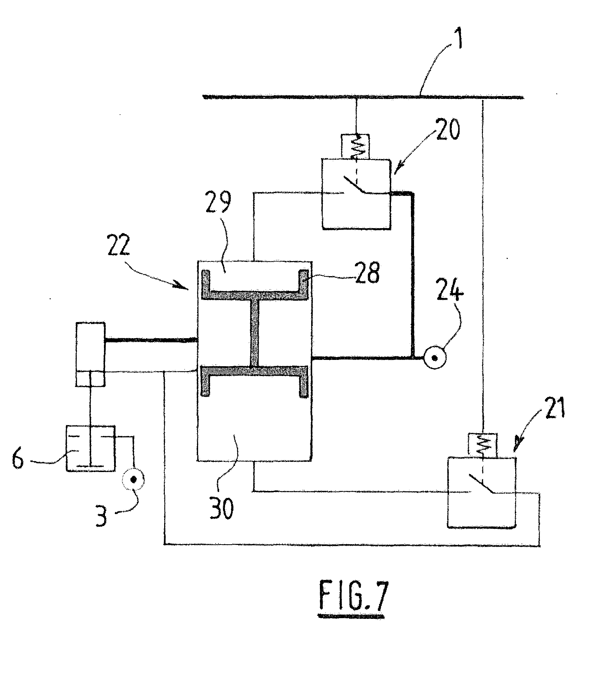

Lorsque cette pression p remonte au-dessus d'une valeur de pression de -550 mbar, le second vacuostat 21 est désactivé, le premier vacuostat 20 restant désactivé. La position haute du piston 28 est maintenue de sorte que l'installation ne produise pas de vide. Cette étape est référencée E sur le diagramme et est représentée sur la figure 7.

Etant donné que la production de vide n'a toujours pas démarrée et que le vide continue d'être consommé, la pression p à l'intérieur du réseau de vide 1 augmente jusqu'à atteindre la valeur de -300 mbar. Le cycle décrit précédemment est donc bouclé, la phase A faisant alors suite à la phase E et la production de vide étant à nouveau assurée par l'installation lors de la phase A.

La production de vide est donc commandée simplement, en fonction de la pression p dans le réseau de vide 1.

Pour une pompe à vide 8 d'un débit de 100 m3/h, le débit d'air consommé est de 216 m³/h, soit avec une autonomie de 90 m3, un temps de production de vide en continu de 25 minutes. Etant donné que, comme vu précédemment, la production de vide n'est pas assurée de manière continue, le temps de fonctionnement réel de l'installation de secours est tout à fait acceptable et permet de pallier sans risque à une panne temporaire.

Comme il va de soi, l'invention ne se limite pas aux seules formes de réalisation de cette installation, décrites ci-dessus à titre d'exemples, mais elle embrasse au contraire toutes les variantes.

## Revendications

1. Installation de secours de production de vide, destinée à être raccordée à un réseau de vide (1) d'un hôpital en cas de panne d'une installation principale de production de vide, qui comporte une pompe à vide (14) comportant une entrée d'aspiration destinée à être raccordée au réseau de vide (1), la pompe à vide (14) étant couplée à un moteur pneumatique (8) raccordé à des moyens de stockage d'air (2) conçus pour délivrer de l'air en cas de panne de manière à actionner le moteur pneumatique (8), et **caractérisée en ce qu'**elle comporte un élément d'aspiration de type venturi (9) possédant une branche d'écoulement principale, raccordée à la sortie de refoulement du moteur pneumatique (8), et une branche d'aspiration raccordée à la pompe à vide.

2. Installation selon la revendication 1, **caractérisée en ce qu'**elle comporte des moyens de réglage (6, 12) du débit d'air délivré au moteur pneumatique (8), par exemple une vanne commandée (6), disposés sur une conduite d'alimentation reliant les moyens de stockage d'air au moteur pneumatique, les moyens de réglage (6, 12) étant commandés en fonction de la pression (p) à l'intérieur du réseau de vide (1).

3. Installation selon la revendication 2, **caractérisée en ce que** les moyens de réglage (12) comportent un premier et un second vacuostats (20, 21) coopérant avec une mémoire pneumatique (22), les moyens de réglage étant conçus pour ouvrir une vanne de réglage (6) du débit d'air dirigé vers le moteur pneumatique (8) lorsque la pression à l'intérieur du réseau de vide (1) est supérieure à une valeur haute limite de pression et pour fermer la vanne de réglage (6) lorsque la pression (p) à l'intérieur du réseau de vide (1) est inférieure à une valeur basse limite de pression.

4. Installation selon la revendication 3, **caractérisée en ce que** la valeur haute limite de pression est de l'ordre de -300 mbar et la valeur basse limite de pression est de l'ordre de -600 mbar.

5. Installation selon l'une des revendications 2 à 4, **caractérisée en ce que** les moyens de réglage (12) sont alimentés en air par les moyens de stockage d'air (2, 24a) et/ou par un réseau d'air distinct (27, 24b).

6. Système de production de vide comportant une installation principale de production de vide, destinée à produire du vide lors du fonctionnement normal du système, de manière à fournir du vide dans un réseau de vide (1), **caractérisé en ce qu'**il comporte une installation de secours selon l'une des revendications 1 à 5, conçue pour produire du vide dans le réseau de vide (1), en cas de panne de l'installation principale de production de vide.

## Claims

1. A vacuum-generating backup plant, to be connected to a vacuum network (1) of a hospital in case of a breakdown of a main vacuum-generating plant, comprising a vacuum pump (14) having a suction inlet to be connected to the vacuum network (1), the vacuum pump (14) being coupled to an air motor (8) connected to air storage means (2) designed to distribute air in case of a breakdown so as to operate the air motor (8), and **characterized in that** it comprises a venturi-like suction element (9) having a main stream branch, connected to the delivery outlet of the air motor (8), and a suction branch connected to the vacuum pump.

2. The plant according to claim 1, **characterized in that** it comprises means (6, 12) for regulating the airflow distributed to the air motor (8), e.g. a controlled valve (6), arranged on a supply duct connecting the air storage means to the air motor, with the regulating means (6, 12) being controlled depending on the pressure (p) inside the vacuum network (1).

3. The plant according to claim 2, **characterized in that** the regulating means (12) comprise a first and a second vacuum switch (20, 21) cooperating with a pneumatic memory (22), the regulating means being designed to open a valve (6) for regulating the airflow directed to the air motor (8) when the pressure inside the vacuum network (1) is greater than an upper pressure limit value, and to close the regulating valve (6) when the pressure (p) inside the vacuum network (1) is lesser than a lower pressure limit value.

4. The plant according to claim 3, **characterized in that** the upper pressure limit value is on the order of -300 mbar and the lower pressure limit value is on the order of -600 mbar.

5. The plant according to any of claims 2 to 4, **characterized in that** the regulating means (12) are supplied with air by the air storage means (2, 24a) and/or a separate air network (27, 24b).

6. A vacuum-generating system comprising a main vacuum-generating system for generating a vacuum during normal operation of the system, so as to provide a vacuum inside a vacuum network (1), **characterized in that** it comprises a backup plant according to any of claims 1 to 5, designed to generate a vacuum inside the vacuum network (1) in case of a breakdown of the main vacuum-generating plant.

## Patentansprüche

1. Ersatz-Vakuumerzeugungsanlage, die dazu gedacht ist, im Falle einer Panne einer Haupt-Vakuumerzeugungsanlage an ein Vakuumnetz (1) eines Krankenhauses angeschlossen zu werden, umfassend eine Vakuumpumpe (14), die einen Ansaugeinlass umfasst, der dazu gedacht ist, an das Vakuumnetz (1) angeschlossen zu werden, wobei die Vakuumpumpe (14) mit einem Druckluftmotor (8) gekoppelt ist, der Luftspeichermittel (2) angeschlossen ist, die dazu ausgelegt sind, um im Falle einer Panne Luft abzugeben, um den Druckluftmotor (8) zu betätigen, und **dadurch gekennzeichnet, dass** sie ein Ansaugelement nach Art eines Venturis (9) umfasst, das einen Hauptströmungszweig, der an den Förderauslass des Druckluftmotors (8) angeschlossen ist, und einen Ansaugzweig, der an die Vakuumpumpe angeschlossen ist, besitzt.

2. Anlage nach Anspruch 1, **dadurch gekennzeichnet, dass** sie Mittel (6, 12) zum Einstellen des Luftdurchsatzes, der an den Druckluftmotor (8) abgegeben wird, z.B. ein gesteuertes Ventil (6), umfasst, die auf einer Versorgungsleitung angeordnet sind, welche die Luftspeichermittel mit dem Druckluftmotor verbindet, umfasst, wobei die Einstellmittel (6, 12) in Abhängigkeit von dem Druck (p) innerhalb des Vakuumnetzes (1) gesteuert werden.

3. Anlage nach Anspruch 2, **dadurch gekennzeichnet, dass** die Einstellmittel (12) einen ersten und einen zweiten Vakuumschalter (20, 21) umfassen, die mit einem Druckluftspeicher (22) zusammenwirken, wobei die Einstellmittel dazu ausgelegt sind, ein Ventil (6) zum Einstellen des Luftflusses, der auf den Druckluftmotor (8) gerichtet wird, zu öffnen, wenn der Druck im Innern des Vakuumnetzes (1) größer ist als ein oberer Druckgrenzwert, und das Regelventil (6) zu verschließen, wenn der Druck (p) im Innern des Vakuumnetzes (1) kleiner als ein unterer Druckgrenzwert ist.

4. Anlage nach Anspruch 3, **dadurch gekennzeichnet, dass** der obere Druckgrenzwert ungefähr -300 mbar und der untere Druckgrenzwert ungefähr -600 mbar beträgt.

5. Anlage nach einem der Ansprüche 2 bis 4, **dadurch gekennzeichnet, dass** die Einstellmittel (12) über die Luftspeichermittel (2, 24a) und/oder ein separates Luftnetz (27, 24b) mit Luft versorgt werden.

6. Vakuumerzeugungssystem, die ein Haupt-Vakuumerzeugungssystem umfasst und dazu gedacht ist, während des normalen Betriebs des Systems ein Vakuum zu erzeugen, um ein Vakuum in einem Vakuumnetz (1) bereitzustellen, **dadurch gekennzeichnet, dass** es eine Ersatzanlage nach einem der Ansprüche 1 bis 5 umfasst, die dazu ausgelegt ist, im Falle einer Panne der Haupt-Vakuumerzeugungsanlage ein Vakuum in dem Vakuumnetz (1) zu erzeugen.
